(19) **Europäisches Patentamt**

**European Patent Office**

**Office européen des brevets**

(11) **EP 0 631 136 B1**

(12) **EUROPÄISCHE PATENTSCHRIFT**

(45) Veröffentlichungstag und Bekanntmachung des Hinweises auf die Patenterteilung:
**02.11.2000 Patentblatt 2000/44**

(51) Int. Cl.$^7$: **G01N 33/36**, D01H 13/22

(21) Anmeldenummer: **94108993.0**

(22) Anmeldetag: **13.06.1994**

(54) **Vorrichtung zur Messung der Masse oder des Substanzquerschnitts von Faserbändern und Verwendung der Vorrichtung**

Apparatus for measuring the mass or cross-sectional density of fibre bands and use of the apparatus

Appareil pour la mesure de la masse ou de la densité transversale de rubans de fibres textiles, et utilisation de l'appareil

(84) Benannte Vertragsstaaten:
**DE ES GR IT PT**

(30) Priorität: **23.06.1993 CH 188693**

(43) Veröffentlichungstag der Anmeldung:
**28.12.1994 Patentblatt 1994/52**

(73) Patentinhaber: **ZELLWEGER LUWA AG**
**8610 Uster (CH)**

(72) Erfinder:
• **Baechler, François**
**CH-8615 Wermatswil (CH)**
• **Strehler, Klaus**
**CH-8048 Zürich (CH)**
• **Harzenmoser, Isidor**
**CH-8304 Wallisellen (CH)**
• **Zehr, Jürg**
**CH-8610 Uster (CH)**

(74) Vertreter: **Ellenberger, Maurice**
**Zellweger Luwa AG**
**Wilstrasse 11**
**8610 Uster (CH)**

(56) Entgegenhaltungen:
**EP-A- 0 401 600**      **GB-A- 2 210 909**
**US-A- 3 435 673**      **US-A- 4 864 853**
**US-A- 5 014 395**

**Beschreibung**

Die vorliegende Erfindung betrifft eine Vorrichtung zur Messung der Masse oder des Substanzquerschnitts von Faserbändern, mit einem Einlaufteil für das zu messende Faserband und mit einem an den Einlaufteil anschliessenden Messteil mit einem Messorgan, das einen das Faserband mechanisch abtastenden meßbacken aufweist,

Vorrichtungen dieser Art werden für Systeme zur Ausregulierung von Bandgewichtsschwankungen und zur Qualitätsdatenerfassung an Faserband produzierenden Maschinen verwendet und dienen dazu, die Garnfeinheitsschwankungen so klein zu halten, dass sie in der Fertigware nicht stören. Die Hauptunterschiede der bekannten Reguliersysteme liegen beim Messorgan, für welches im wesentlichen drei Typen, das sogenannte aktiv-pneumatische Messorgan, das Walzenmesssystem und das Faserpressungssystem bekannt sind. Bezüglich der ersten beiden Messorgane wird auf das USTER News Bulletin Nr. 30, 1982, verwiesen, bezüglich des letzgenannten Messorgans auf die US-A-4 864 853, welche den, dem Gegenstand der Anmeldung am nächsten kommenden, Stand der Technik aufzeigt.

Aus der US 3,435,673 ist eine Vorrichtung zur Messung des Substanzquerschnittes von textilem Bandmaterial bekannt, bei der das Band durch eine Düse mit einer Verengung hindurchgezogen wird. Der pneumatische Druck, der in der Verengung gemessen wird, soll dem Substanzquerschnitt des textilen Bandmaterials entsprechen.

Den verschiedenen Messorganen ist gemeinsam, dass sie einerseits neben der Abhängigkeit von der Faserbandmasse noch weitere, zumeist vernachlässigbare, textiltechnisch relevante Abhängigkeiten aufweisen, und andererseits aber nur die Messung der Faserbandmasse ermöglichen. Wenn eine weitere Eigenschaft des Faserbandes überwacht oder gemessen werden soll, dann ist der Einsatz eines zusätzlichen Messorgans erforderlich, der aber aus Platzgründen in der Regel an einer anderen Messstelle erfolgen muss. Durch die Erfindung soll nun eine Vorrichtung der eingangs genannten Art angegeben werden, bei der jeder Einfluss von Nebengrössen ausser der Faserbandmasse ausgeschaltet ist, und die aber trotzdem Rückschlüsse auf diese Nebengrössen zulässt.

Diese Aufgabe wird erfindungsgemäss dadurch gelöst, dass am Messteil ein zweites nach einem unterschiedlichen Messprinzip arbeitendes Messorgan für die Faserbandmasse vorgesehen ist, das ein Mittel zur Messung des durch das Faserband an einer Verengung bewirkten pneumatischen Drucks aufweist.

Dadurch, dass zwei Messorgane für ein und dieselbe Grösse verwendet werden, erhält das Messsystem Redundanz, was einerseits die Ausschaltung des Einflusses der genannten Nebengrössen und andererseits eine gewisse Selbstkontrolle des Messsystems ermöglicht. Durch die Verwendung der beiden genannten Messorgane wird es möglich, zu einer Aussage über die genannten Nebengrössen zu kommen. Da die beiden Messorgane an praktisch ein und derselben Messstelle eingebaut werden können, ist die Vergleichbarkeit der Messergebnisse sichergestellt, was für die Ausschaltung des Einflusses der Nebengrössen wichtig ist. Hinsichtlich der Aussagen über die Nebengrössen ist zu bemerken, dass diese in der Regel nicht quantitative Messergebnisse eines bestimmten Parameters sondern ein Mass für eine Kenngrösse darstellen werden, die online eine Aussage über die Zusammensetzung des untersuchten Faserbandes liefert.

Die Erfindung betrifft weiter eine Verwendung der genannten Vorrichtung zur Gewinnung einer Kenngrösse für die Zusammensetzung des erfassten Faserbandes. Diese Verwendung ist dadurch gekennzeichnet, dass die Signale der beiden Messorgane auf für bestimmte, die Zusammensetzung beeinflussende Parameter charakteristische gegenseitige Abweichungen untersucht werden.

Die Erfindung betrifft ausserdem eine Verwendung der genannten Vorrichtung zur getrennten Regulierung und Ueberwachung in einer Regelstrecke. Diese Verwendung ist dadurch gekennzeichnet, dass das Signal des einen, vorzugsweise des aktiv-pneumatischen Messorgans, für die Bandregulierung und das Signal des anderen, vorzugsweise des Faserpressungs-Messorgans, für die Bandüberwachung verwendet wird.

Nachfolgend wird die Erfindung anhand von Ausführungsbeispielen und der Zeichnungen näher erläutert; es zeigt:

Fig. 1    eine Perspektivdarstellung eines ersten Ausführungsbeispiels einer erfindungsgemässen Vorrichtung,
Fig. 2    einen achsialen Längsschnitt durch das Ausführungsbeispiels von Fig. 1,
Fig. 3    eine Perspektivdarstellung eines zweiten Ausführungsbeispiels einer erfindungsgemässen Vorrichtung,
Fig. 4    einen achsialen Längsschnitt durch das Ausführungsbeispiel von Fig. 3; und
Fig. 5    ein Prinzip-Schema einer Streckenregulierung mit einer erfindungsgemässen Vorrichtung als Messorgan.

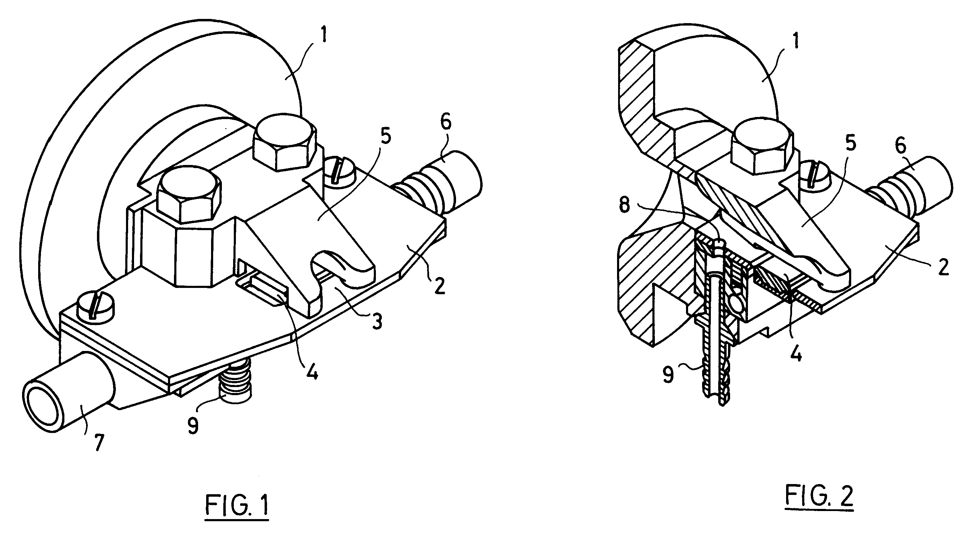

Das in den Figuren 1 bis 4 dargestellten Messorgan entspricht im wesentlichen der in der US-A-4 864 853, auf deren Offenbarung hiermit ausdrücklich Bezug genommen wird, beschriebenen Vorrichtung. Das Messorgan enthält in bekannter Weise einen Einlaufteil 1 für die Vorverdichtung des Faserbandes und einen an diesem befestigten Messteil 2 mit einem Messkanal 3, der durch eine Messebene und durch eine rinnenförmige Bandführung begrenzt ist. Die Messebene enthält einen durch eine mit Dehnungsmessstreifen bestückte Blattfeder gebildeten Messbalken 4 und die Bandführung ist aus einem auf dem Messteil 2 auswechselbar befestigten Führungsteil 5 herausgearbeitet.

Der Messteil 2 enthält ausserdem einen Anschlussnippel 6 für die Kühlung und Reinigung des Messteils durch Pressluft, wobei die Kühlung dauernd und die Reinigung intermittierend erfolgt. Mit dem Bezugszeichen 7 ist ein elektrischer Anschluss für die Erfassung des Signals des Messbalkens 4 bezeichnet.

Alle bis jetzt erwähnten Teile sind von dem in der genannten US-A-4 864 853 beschriebenen Faserpressungs-Messsystem bekannt, dessen Sensor durch den Messbalken 4 gebildet ist. Das dargestellte Messorgan weist nun zusätzlich noch ein aktiv-pneumatisches Messorgan der in der US-A-3 435 673 beschriebenen Art auf, mit welchem der durch das bewegte Faserband an einer Verengung bewirkte Druck als Messgrösse ausgewertet wird. Die Verengung befindet sich im Bereich zwischen dem Einlaufteil 1 und dem Messbalken 4 und die Mittel zur Auswertung des Drucks umfassen eine Bohrung 8 in der betreffenden Wand des Führungskanals 3 und einen von dieser Bohrung wegführenden Anschluss für die Erfassung des aktiv-pneumatischen Messsignals.

Die beiden Ausführungsformen der Fig. 1, 2 und 3, 4 unterscheiden sich dadurch, dass die Bohrung 8 im ersten Fall in der den Messbalken 4 enthaltenden Messebene und im zweiten Fall in dem dem Messbalken 4 gegenüberliegenden Führungsteil 5 angebracht ist.

In der folgenden Funktionsbeschreibung wird zur Kennzeichnung der Funktion und der Elemente des Faserpressungs-Messsystems (Messkanal 3, Messbalken 4, Führungsteil 5) die Bezeichnung FP und zur Kennzeichnung der Funktion und der Elemente des aktivpneumatischen Messsystems (Messkanal 3, Bohrung 8, Anschluss 9) die Bezeichnung AP verwendet.

Der Messbalken 4 (den man auch als Messmembran bezeichnen könnte) des FP-Messteils liefert ein Signal in Funktion der Kompression des Faserbandes im Messkanal 3. Dieses Signal wird durch einen an den Anschluss 7 angeschlossenen Vorverstärker verstärkt, woraus sich das FP-Messsignal $S_{FP}$ ergibt, welches eine Funktion des Messkanalquerschnitts $Q_k$, der Faserbandmasse $B_m$, der Bandgeschwindigkeit $B_v$, und der Faserband-Zusammensetzung $B_z$, ist:

$$S_{FP} = f(Q_k, B_m, B_v, B_z)$$

Mit der Grösse des Messkanalquerschnitts wird die optimale Bedingung der FP-Messfunktion in Funktion von der mittleren Faserbandmasse bestimmt. Bei konstanter Bandgeschwindigkeit $B_v$ und korrektem Messkanalquerschnitt $Q_k$ variiert das FP-Signal nach folgender Gleichung:

$$\delta S_{FP} = K_{kpm} \cdot \delta B_m + K_{fpz} \cdot \delta B_z \qquad (\delta X \equiv \Delta X / X)$$

$K_{fpm}$ : FP-Umwandlungsfaktor der Faserbandmasse
$K_{fpz}$ : FP-Umwandlungsfaktor der Faserband-Zusammensetzung

Die Kompression des Faserbandes im Messkanal erzeugt einen pneumatischen Druck, der über die Bohrung 8 am Anschluss 9 erfasst und durch einen Druckwandler in ein elektrisches Signal $S_{AP}$ umgewandelt wird. Dieses Signal ist eine Funktion des Messkanalquerschnitts $Q_k$, der Faserbandmasse $B_m$, Faserbandgeschwindigkeit $B_v$ und der Faserfeinheit $B_f$:

$$S_{AP} = f(Q_k, B_m, B_v, B_f)$$

Bei konstanter Bandgeschwindigkeit $B_v$ und mit dem für die FP-Funktion verwendeten Messkanalquerschnitt $Q_k$ variiert das AP-Signal nach folgender Gleichung:

$$\delta S_{AP} = K_{apm} \cdot \delta B_m + K_{apf} \cdot \delta B_f$$

$K_{apm}$: AP-Umwandlungsfaktor der Faserbandmasse
$K_{apf}$: AP-Umwandlungsfaktor der Faserfeinheit

Somit liefern die beiden Signale des Messteils 2 Angaben über drei verschiedene Eigenschaften eines Faserbandes, das sind

- die Faserbandmassen-Variationen $\delta B_m$
- die Faserfeinheits-Variationen $\delta B_f$
- die Faserband-Zusammensetzungs-Variationen $\delta B_z$.

Durch die Serienschaltung der beiden unabhängigen und verschiedenartigen Messsysteme an einer Messstelle wird es also möglich, neben der Messung der Faserbandmasse eine in einer Kenngrösse zusammengefasste Aussage über weitere textiltechnische Parameter, insbesondere über Faserfeinheit und Faserparallelisierung, zu machen. Diese Kenngrösse, in die noch weitere Parameter, wie Faserlänge, Bauschigkeit, Kurzfaseranteil, Anteil schwimmender Fasern, und spezifisches Gewicht, eingehen, liefert einen qualitativen Hinweis auf die Zusammensetzung des erfassten Faserbandes.

So ermöglicht die Serieschaltung der genannten Messsysteme beispielsweise die optimale Einstellung der Streckwerks-Distanz, da eine Zunahme von schwimmenden Fasern ein charakteristisches Ansteigen des FP-Messsignals bewirkt.

| Fall | Signale | | Faserband | | | Wahr-schein-lichkeit |
|---|---|---|---|---|---|---|
| | $\delta S_{FP}$ | $\delta S_{AP}$ | $\delta B_m$ | $\delta B_f$ | $\delta B_z$ | |
| 1 | 0 | 0 | 0 | 0 | 0 | klein |
| 2 | 0 | $\neq 0$ | 0 | $\neq 0$ | 0 | gross |
| 3 | $\neq 0$ | 0 | 0 | 0 | $\neq 0$ | klein |
| 4 | $\neq 0$  $\neq 0$ ; $x = y$ | | $\neq 0$ | 0 | 0 | sehr gross |
| 5 | $\neq 0$  $\neq 0$ ; $x = y$ | | 0 oder $\neq 0$ | $\neq 0$  $Q = R$ | $\neq 0$ | sehr klein |
| 6 | $\neq 0$  $\neq 0$ ; $x \neq y$ | | $\neq 0$ | $\neq 0$  $\neq \delta B_z$ | $\neq 0$  $\neq \delta B_f$ | mittel |

Die Auswertung der FP/AP-Signale kann nach dem in der vorstehenden Tabelle angegebenen Schema erfolgen, wobei für die verschiedenen Fälle des FP/AP-Signals jeweils angegeben ist, von welcher der drei genannten Eigenschaften eine Variation vorliegen könnte und wie hoch die Wahrscheinlichkeit dafür ist. In den Fällen 1 bis 4 können die verschiedenen Abweichungen eindeutig erkannt und lokalisiert werden.

In der vorstehenden Tabelle bezeichnen die bei den Fällen 4 bis 6 verwendeten Symbole X und Y jeweils den Quotienten $\delta S_{AP}/K_{apm}$ beziehungsweise $\delta S_{FP}/K_{fpm}$; die bei Fall 5 verwendeten Symbole Q und R bezeichnen die Produkte $\delta B_p \cdot K_{fpp}$ beziehungsweise $\delta B_f \cdot K_{apf}$.

Das beschriebene kombinierte Messorgan (Kombi-Messorgan) kann einerseits als Messorgan zur Bestimmung der Qualitätsdaten eines Faserbandes eingesetzt werden und andererseits als Messorgan für die Regulierung und zur Vergleichmässigung der Masse eines Faserbandes. Im letzteren Fall werden Ungleichmässigkeiten nicht nur registriert sondern lösen entsprechende Regelvorgänge aus (siehe dazu beispielsweise das USTER News Bulletin Nr. 30, 1982). Da die bekannten Messorgane nicht nur auf Veränderungen der Faserbandmasse ansprechen, sondern wie gezeigt wurde, auch auf solche der Faserfeinheit (AP-Messorgan) oder der Faserband-Zusammensetzung (FP-Messorgan), muss neben dem Messorgan noch ein zusätzliches Ueberwachungsorgan zur Kontrolle der Regelung verwendet werden. Denn wenn beispielsweise ein AP-Messorgan verwendet wird, dann würde bei diesem eine Aenderung der Faserfeinheit einen Regulierungsschritt in Richtung Veränderung der Faserbandmasse bewirken, was nicht nur falsch wäre, sondern vom Messorgan auch gar nicht bemerkt würde. Bei Verwendung des beschriebenen Kombi-Messorgans mit dem AP- und mit dem FP-Messorgan kann dagegen auf das zusätzliche Ueberwachungsorgan verzichtet werden.

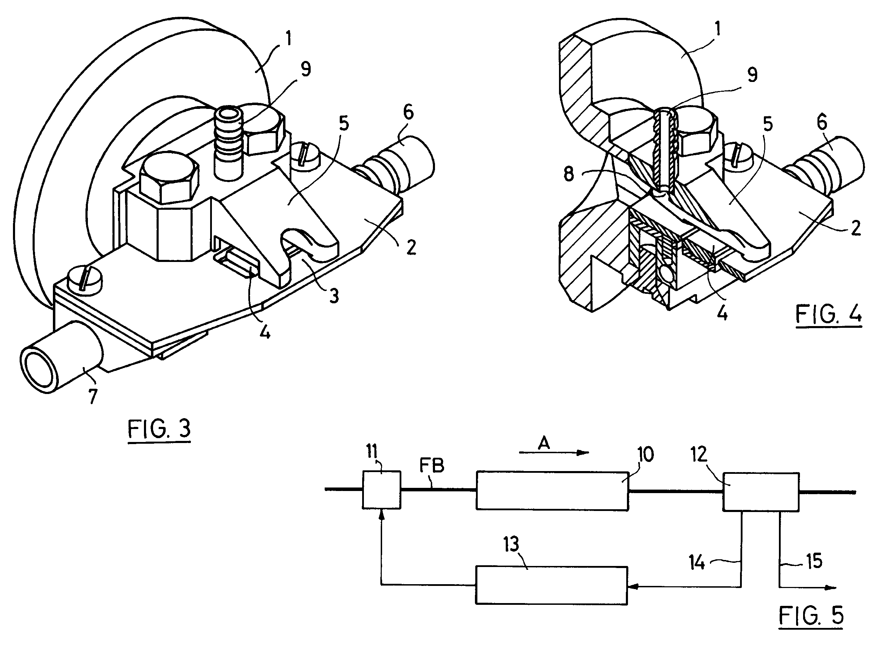

Fig. 5 zeigt ein Prinzischema einer mit einem Kombi-Messorgan ausgerüsteten Streckenregulierung, die in Richtung des Pfeiles A von einem Faserband FB durchlaufen wird. Die Streckenregulierung besteht im wesentlichen aus einer Regelstrecke 10, aus einem dieser vorgeschalteten Stellglied 11, aus einem der Regelstrecke 10 nachgeschalteten Kombi-Messorgan 12 und aus einem zwischen Messorgan 12 und Stellglied 11 angeordneten Regler 13.

Wenn das Faserband FB eine Abweichung (Störgrösse) aufweist, dann wird diese vom Kombi-Messorgan 12 festgestellt, das an den Regler 13 über eine Leitung 14 ein entsprechendes Signal für die Bandregulierung abgibt. Der Regler seinerseits berechnet die erforderliche Regelgrösse und steuert das Stellglied 11 entsprechend an. Das Signal für die Bandregulierung kann beispielsweise das AP-Messsignal sein. Gleichzeitig dient das zweite Messorgan des Kombi-Messorgans 12, in diesem Fall das FP-Messorgan, für die Bandüberwachung und liefert auf einer Leitung 15 ein entsprechendes Signal.

Das beschriebene Kombi-Messorgan hat die folgenden Hauptmerkmale:

- Kontinuierliche Messung der Faserbandmasse durch zwei verschiedene Messmethoden, die auf weitere Parameter wie Faserfeinheit und Faserband-Zusammensetzung verschieden ansprechen.
- Der Vergleich der mit den beiden Messmethoden gewonnenen Signale ermöglicht einerseits eine Selbstkontrolle des Messorgans und liefert andererseits eine Kenngrösse, die zumindest eine qualitative Aussage über die Zusammensetzung des erfassten Faserbandes zulässt.
- Möglichkeit des Einsatzes des Kombi-Messorgans für die getrennten Funktionen Regulierung und Ueberwachung.
- Durch Auswechseln des Führungsteils 5 am Kombi-Messorgan können sowohl für die FP- als auch für die AP-Funktion die optimalen Arbeitsbedingungen für das jeweilige Faserband bestimmt werden.

**Patentansprüche**

1.   Vorrichtung zur Messung der Masse oder des Substanz-querschnitts von Faserbändern, mit einem Einlaufteil (1) für das zu messende Faserband und mit einem an den Einlaufteil anschliessenden Messteil (2) mit einem Messorgan, das einen das Faserband (FB) mechanisch abtastenden Messbalken (4) aufweist, dadurch gekennzeichnet, dass am Messteil (2) ein zweites nach einem unterschiedlichen Messprinzip arbeitendes Messorgan für die Faserbandmasse vorgesehen ist, das ein Mittel (8, 9) zur Messung des durch das Faserband (FB) an einer Verengung bewirkten pneumatischen Druckes aufweist.

2.   Vorrichtung nach Anspruch 1, dadurch gekennzeichnet, dass der Messteil (2) einen Messkanal (3) aufweist, der durch eine Fläche und eine rinnenförmige Bandführung begrenzt ist, wobei die Fläche zum Messbalken (4) des einen Messorgans gehört, das das Faserband (FB) mechanisch abtastet und dass das Messorgan (8, 9) mit Mitteln zur Messung des pneumatischen Druckes in diesem Messkanal angeordnet ist.

3.   Vorrichtung nach Anspruch 2, dadurch gekennzeichnet, dass das Messorgan (8, 9) mit Mitteln zur Messung des pneumatischen Druckes, in der den Messbalken (4) enthaltenden Fläche des Messkanals (3) angeordnet ist.

4.   Vorrichtung nach Anspruch 2, dadurch gekennzeichnet, dass das Messorgan (8, 9) mit Mitteln zur Messung des pneumatischen Druckes in einem rinnenförmigen Bandführungsteil des Messkanals (3) angeordnet ist, der, der den Messbalken (4) enthaltenden, Fläche gegenüberliegt.

5.   Vorrichtung nach Anspruch 3 oder 4, dadurch gekennzeichnet, dass die von den beiden Messorganen (4; 8, 9) gewonnenen Signale einer gemeinsamen Auswertungsstufe zugeführt sind, in der ein Vergleich der beiden Signale erfolgt.

6.   Verwendung der Vorrichtung nach Anspruch 1 zur Gewinnung einer Kenngrösse für die Zusammensetzung des erfassten Faserbandes, wobei die Signale der beiden Messorgane (4; 8, 9) auf für bestimmte, die Zusammensetzung beeinflussende Parameter charakteristische gegenseitige Abweichungen untersucht werden.

7.   Verwendung nach Anspruch 6, dadurch gekennzeichnet, dass charakteristische Abweichungen des Signals des Messorgans (8, 9), mit Mitteln zur Messung des pneumatischen Druckes, als Hinweis auf Schwankungen der Faserfeinheit interpretiert werden.

8.   Verwendung nach Anspruch 6, dadurch gekennzeichnet, dass charakteristische Abweichungen des Signals des Messorgans (4), das einen das Faserband (FB) mechanisch abtastenden Messbalken (4) aufweist, als Hinweis auf Schwankungen der Faserband-Zusammensetzung interpretiert werden.

9.   Verwendung nach Anspruch 6, dadurch gekennzeichnet, dass charakteristische Abweichungen des Signals des Messorgans (4), das einen das Faserband (FB) mechanisch abtastenden Messbalken (4) aufweist, als Hinweis auf sogenannte "schwimmende" Fasern interpretiert und dass derartige Hinweise zur Einstellung der optimalen Streckwerkdistanz von Verzugsstellen an bänderproduzierenden Maschinen verwendet werden.

**10.** Verwendung der Vorrichtung nach Anspruch 1 zur getrennten Regulierung und Ueberwachung in einer Regelstrecke, wobei das Signal des einen Messorgans (8, 9), für die Bandregulierung und das Signal des anderen, Messorgans (4), für die Bandüberwachung verwendet wird.

**Claims**

**1.** Device for measuring the mass or substance cross-section of slivers, with an entry part (1) for the sliver to be measured and with a measuring part (2), adjoining the entry part, with a measuring element which comprises a measuring bar (4) which mechanically samples the sliver (FB), characterized in that there is provided on the measuring part (2) a second measuring element for the sliver mass, operating according to a different measurement principle, which comprises a means (8, 9) for measuring the pneumatic pressure caused by the sliver (FB) at a contraction.

**2.** Device according to Claim 1, characterized in that the measuring part (2) comprises a measuring channel (3) which is delimited by a face and a grooved sliver guide, the face belonging to the one measuring bar (4) which mechanically samples the sliver (FB), and in that the measuring element (8, 9) is disposed with means for measuring the pneumatic pressure in this measuring channel.

**3.** Device according to Claim 2, characterized in that the measuring element (8, 9) is disposed with means for measuring the pneumatic pressure, in the face of the measuring channel (3) containing the measuring bar (4).

**4.** Device according to Claim 2, characterized in that the measuring element (8, 9) is disposed with means for measuring the pneumatic pressure in a grooved sliver-guide part of the measuring channel (3) located opposite the face containing the measuring bar (4).

**5.** Device according to Claim 3 or 4, characterized in that the signals obtained from the two measuring elements (4; 8, 9) are fed to a common evaluation stage in which comparison of the two signals is performed.

**6.** Use of the device according to Claim 1 for obtaining a characteristic quantity for the composition of the recorded sliver, the signals of the two measuring elements (4; 8, 9) being examined for mutual deviations characteristic of specific parameters influencing the composition.

**7.** Use according to Claim 6, characterised in that characteristic deviations of the signal of the measuring element (8, 9) are interpreted, with means for measuring the pneumatic pressure, as an indication of fluctuations in the fibre fineness.

**8.** Use according to Claim 6, characterized in that characteristic deviations of the signal of the measuring element (4) which comprises a measuring bar (4) which mechanically samples the sliver (FB) are interpreted as an indication of fluctuations in the sliver composition.

**9.** Use according to Claim 6, characterized in that characteristic deviations of the signal of the measuring element (4) which comprises a measuring bar (4) which mechanically samples the sliver (FB) are interpreted as an indication of so-called "floating" fibres, and in that such indications are used for setting the optimum drafting-unit distance of drafting points on sliver-producing machines.

**10.** Use of the device according to Claim 1 for separate regulation and monitoring in a control stage, the signal of one measuring element (8, 9) being used for sliver regulation and the signal of the other measuring element (4) being used for sliver monitoring.

**Revendications**

**1.** Dispositif pour mesurer la masse ou la section transversale de la substance de rubans de fibres, avec une partie d'entrée (1) pour le ruban de fibres à mesurer et avec une partie de mesure (2) faisant suite à la partie d'entrée, avec un organe de mesure qui présente une poutre de mesure (4) tâtant mécaniquement le ruban de fibres (FB), caractérisé en ce qu'il est prévu à la partie de mesure (2) un deuxième organe de mesure pour la masse du ruban de fibres fonctionnant selon un principe de mesure différent, qui comporte un moyen (8, 9) pour mesurer la pression pneumatique provoquée par le ruban de fibres (FB) à un retrécisssement.

**2.** Dispositif selon la revendication 1, caractérisé en ce que la partie de mesure (2) présente un canal de mesure (3) qui est délimité par une face et un guidage de ruban en forme de rainure, où la face fait partie de la poutre de mesure (4) de l'organe de mesure précité, qui tâte mécaniquement le ruban de fibres (FB), et que l'organe de mesure (8, 9) comportant des moyens pour mesurer la pression pneumatique est disposé dans ce canal de mesure.

3. Dispositif selon la revendication 2, caractérisé en ce que l'organe de mesure (8, 9), avec les moyens pour mesurer la pression pneumatique, est disposé dans la face du canal de mesure (3) contenant la poutre de mesure (4).

4. Dispositif selon la revendication 2, caractérisé en ce que l'organe de mesure (8, 9) comportant des moyens pour mesurer la pression pneumatique est disposé dans une partie de guidage de ruban en forme de rainure du canal de mesure (3) qui est opposée à la face contenant la poutre de mesure (4).

5. Dispositif selon la revendication 3 ou 4, caractérisé en ce que les signaux obtenus par les deux organes de mesure (4; 8, 9) sont amenés à un étage d'évaluation commun dans lequel a lieu une comparaison des deux signaux.

6. Utilisation du dispositif selon la revendication 1 pour obtenir une grandeur caractéristique de la composition du ruban de fibres détecté, où les signaux des deux organes de mesure (4; 8, 9) sont examinés en vue d'écarts mutuels caractéristiques de paramètres définis, agissant sur la composition.

7. Utilisation selon la revendication 6, caractérisée en ce que les écarts caractéristiques du signal de l'organe de mesure (8, 9), avec des moyens pour mesurer la pression pneumatique, sont interprétés comme une indication signalant des oscillations de la finesse des fibres.

8. Utilisation selon la revendication 6, caractérisée en ce que des écarts caractéristiques du signal de l'organe de mesure (4), qui présente une poutre de mesure (4) tâtant mécaniquement le ruban de fibres (FB), sont interprétés comme constituant des indications à des oscillations de la composition du ruban de fibres.

9. Utilisation selon la revendication 6, caractérisée en ce que des écarts caractéristiques du signal de l'organe de mesure (4), qui présente une poutre de mesure (4) tâtant mécaniquement le ruban de fibres (FB), sont interprétés comme constituant une indication à des soi-disant fibres "flottantes" et que de telles indications sont utilisées pour régler la distance optimale du dispositif d'étirage d'emplacements d'étirage à des machines produisant des rubans.

10. Utilisation du dispositif selon la revendication 1 pour le réglage et la surveillance séparés dans un régulateur d'étirage, où le signal d'un organe de mesure (8, 9) est utilisé pour le réglage du ruban et le signal de l'autre organe de mesure (4), pour la surveillance du ruban.

FIG. 2

FIG.1

FIG. 3

FIG. 4

FIG. 5

EP 0 631 136 B1